# EUROPÄISCHE PATENTSCHRIFT

(11) **EP 3 581 640 B1**
(45) Veröffentlichungstag und Bekanntmachung des Hinweises auf die Patenterteilung: **22.04.2026**
(21) Anmeldenummer: 19178635.9
(22) Anmeldetag: 06.06.2019
(51) Int. Cl.: C02F 1/04, C02F 1/20, C12M 1/00, C02F 11/13, B01D 1/00, B01D 5/00, B01D 53/00

(54) **VERFAHREN UND VORRICHTUNG ZUR AUFBEREITUNG VON BIOLOGISCHEN STOFFEN, INSBESONDERE VON GÄRRESTEN PFLANZLICHER UND TIERISCHER HERKUNFT, INSBESONDERE AUS BIOGASANLAGEN, VON GÜLLE ODER VON SONSTIGEN ORGANISCHEN ABFALLPRODUKTEN**
METHOD AND DEVICE FOR THE TREATMENT OF BIOLOGICAL SUBSTANCES, ESPECIALLY FOOD REMAINS OF PLANT AND ANIMAL ORIGIN, IN PARTICULAR FROM BIOGAS FACILITIES, LIQUID MANURE OR OTHER ORGANIC WASTE PRODUCTS
PROCÉDÉ ET DISPOSITIF DE PRÉPARATION DES SUBSTANCES BIOLOGIQUES, EN PARTICULIER D'ORIGINE VÉGÉTALE ET D'ORIGINE ANIMALE DE FERMENTATION, EN PARTICULIER DES INSTALLATIONS DE BIOGAZ, À PARTIR DU LISIER OU D'AUTRES PRODUITS DE DÉCHETS ORGANIQUES

(30) Priorität: 15.06.2018 DE 102018114366
(43) Veröffentlichungstag der Anmeldung: 18.12.2019
(73) Patentinhaber: EEO Tech GmbH, 49835 Wietmarschen (DE)
(72) Erfinder: Osseforth, Ewald, 49835 Wietmarschen (DE)
(74) Vertreter: Manasse, Uwe

(56) Entgegenhaltungen:
- WO-A1-2015/067240
- DE-A1- 102014 005 667
- US-A- 5 810 975
- US-A1- 2014 045 234
- US-A1- 2015 329 399
- US-B1- 8 637 304

## Beschreibung

Die vorliegende Erfindung betrifft ein Verfahren und eine Vorrichtung zur Aufbereitung von biologischen Stoffen, insbesondere von Gärresten pflanzlicher und tierischer Herkunft, insbesondere aus Biogasanlagen, von Gülle oder von sonstigen organischen Abfallprodukten. Zu den biologischen Stoffen kann auch Jauche gezählt werden. Gärreste, Gülle etc. werden auch als flüssige Wirtschaftsdünger bezeichnet.

Es gibt mittlerweile diverse Anlagen zur Trocknung von Gärresten etc., wobei aber keine der bekannten Anlagen zufriedenstellende Ergebnisse liefert. Zurzeit wird die Abtrennung von Wasser und Stickstoffverbindungen, wie bspw. Ammoniak (NH₃) oder Ammonium (NH₄⁺)-Salze, realisiert. Dies erfolgt allerdings mit erheblichem Aufwand und/oder mit hohen Kosten. Die Trocknung von Gärresten etc. wird vorzugsweise mit Restwärme von Blockheizkraftwerken unter Anderem aus dem Grund realisiert, weil sie mit einer hohen Subventionierung kombiniert werden kann. Ein großer Nachteil besteht hier im extrem hohen Energieverbrauch von 600-1000 kW, um den Flüssiganteil von 1 m³/h Gärrest zu verdampfen (Trocknung) und dadurch den Gärrest (ca. 7 % der Gärrest-Masse) als Feststoff vorliegen zu haben. Soll zusätzlich eine Ammoniakbehandlung integriert werden, wird eine Stripperanlage bzw. ein Schwefelsäure-Wäscher notwendig. In den meisten Anwendungen wird allerdings immer noch Ammoniak direkt in die Abluft gegeben, was zu einer nicht unwesentlichen Umweltbelastung führt.

DE 69312690 T2 betrifft ein Verfahren, eine Vorrichtung und eine Anlage zur Extraktion durch Verdampfung eines festen Rückstandes aus einem fließfähigen Material.

DE 202014010811 US 1 offenbart eine Vorrichtung zur Trocknung von Gärresten, bestehend aus einem Gärrestetrockner, dieser bestehend aus einem Verdampferkessel mit einem Gärrestezufluss und einem Gärresteabfluss, einem Dampfauslass, einer Heizvorrichtung mit einer Dampf- oder Heißwasserzuleitung und einer Kondensat- oder Heißwasserableitung.

DE 102014005667 A1 offenbart eine Vorrichtung und ein Verfahren zur Trocknung von Gärresten. Zur Trocknung von Gärresten aus Biogasanlagen oder Gülle weist ein Gärrestetrockner einen Verdampfer mit einer Heizvorrichtung auf. Die Heizvorrichtung kann mit Dampf oder Heißwasser beheizt werden. Mit mehreren Gärrestetrocknern lassen sich verschiedene Anlagenkonstellationen für eine effiziente Trocknung unterschiedlicher Gärreste realisieren.

Weiteren lediglich technologischen Hintergrund bilden US 8637304 B1, US 2014045234 A1 und US 2015329399 A1.

Der vorliegenden Erfindung liegt somit die Aufgabe zugrunde, mit relativ wenig Energie Wasser und Stickstoffverbindungen aus einem biologischen Stoff und die Stickstoffverbindungen auch noch von dem Wasser abzutrennen, zumindest aber eine Abreicherung von Stickstoffverbindungen zu erreichen.

Gemäß einem ersten Aspekt wird diese Aufgabe gelöst durch eine Vorrichtung nach Anspruch 1.

Gemäß einem zweiten Aspekt wird diese Aufgabe gelöst durch ein Verfahren nach Anspruch 18.

Bei dem Verfahren kann vorgesehen sein, dass es Auskondensation von gasförmigen Stickstoffverbindungen, insbesondere Ammoniak, nach einer ersten Kondensationsstufe zur Kondensation des Stickstoffverbindungen enthaltenden Brüden in einer zweiten Kondensationsstufe umfasst.

Gemäß einer besonderen Ausführungsform der vorliegenden Erfindung umfasst es Sammeln der auskondensierten Stickstoffverbindungen, insbesondere Ammoniak, in einer Sammeleinrichtung, insbesondere einem Ammoniaksammler.

Alternativ kann vorgesehen werden, dass es Vermischen der auskondensierten Stickstoffverbindungen, insbesondere Ammoniak, mit einer Säure zu Flüssigdünger umfasst.

Insbesondere kann dabei vorgesehen sein, dass es Lagern des Flüssigdüngers zur späteren Nutzung umfasst.

Alternativ kann vorgesehen sein, dass es Kondensation des Stickstoffverbindungen enthaltenden Brüden in einer ersten Kondensationsstufe und Ausleiten von gasförmigen Stickstoffverbindungen, insbesondere Ammoniak, zur Weiterverarbeitung umfasst.

Bei der Vorrichtung kann vorgesehen sein, dass sie eine Steuereinrichtung zur Steuerung des Drehantriebs und/oder der Heizeinrichtung und/oder der Befüllung des Behälters und/oder der Abführung der aufkonzentrierten Flüssigphase und/oder der Gasphase aufweist.

Gemäß einer besonderen Ausführungsform sind zum Erwärmen des biologischen Stoffes im Inneren des Behälters die hohlen zylindrischen Überlaufwände mit Wasser und Dampf befüllt.

Vorteilhafterweise weist die Vorrichtung eine erste Fördereinrichtung zum Abfördern der aufkonzentrierten Flüssigphase auf, die mit der ersten Behälterauslassöffnung in Fluidverbindung steht.

Insbesondere kann dabei vorgesehen sein, dass die erste Fördereinrichtung eine Förderspirale umfasst.

Vorteilhafterweise ist die erste Fördereinrichtung zusätzlich zur thermischen Nachtrocknung der aufkonzentrierten Flüssigphase ausgebildet.

Gemäß einer weiteren besonderen Ausführungsform weist die Vorrichtung eine der ersten Fördereinrichtung nachgeschaltete Torrefizierungseinrichtung zur Torrefizierung des überwiegend festen Produktes der Nachtrocknung auf.

Insbesondere kann dabei vorgesehen sein, dass die Torifizierungseinrichtung eine der ersten Fördereinrichtung nachgeschaltete zweite Fördereinrichtung, insbesondere eine Förderspirale, aufweist.

Gemäß einer weiteren besonderen Ausführungsform weist die Vorrichtung eine Stickstoffverbindungen Abtrenneinrichtung auf, die zur Abtrennung von Stickstoffverbindungen, wie zum Beispiel Ammoniak oder Ammonium, aus der Gasphase mit der zweiten Behälterauslassöffnung direkt oder indirekt in Fluidverbindung steht.

Vorteilhafterweise weist die Stickstoffverbindungen Abtrenneinrichtung eine Separiereinrichtung zur Separierung eines Wasseranteils durch Kondensation von Wasser aus der Gasphase in der radial äußeren oder radial äußersten Überlaufwand auf.

Zweckmäßigerweise weist die Stickstoffverbindungen Abtrenneinrichtung eine Stripping-Einrichtung zum Stickstoffverbindungen-Stripping in der radial inneren Überlaufwand oder in mindestens einer der radial inneren Überlaufwände auf.

Zweckmäßigerweise weist die Vorrichtung eine Stickstoffverbindungen Bindeeinrichtung zur Erzeugung von Flüssigdünger aus den abgetrennten Stickstoffverbindungen auf.

Ebenso kann vorgesehen sein, dass sie eine Ausleiteinrichtung zum Ausleiten von aufkonzentrierten Stickstoffverbindungen zur Weiterverarbeitung aufweist.

Zweckmäßigerweise weist die Vorrichtung eine Wärmerückgewinnungseinrichtung zur Rückgewinnung von Wärmeenergie der Gasphase auf.

Insbesondere kann dabei vorgesehen sein, dass die Wärmerückgewinnungseinrichtung einen Brüdenverdichter aufweist, der mit der zweiten Behälterauslassöffnung zur Verdichtung der Gasphase in Fluidverbindung steht.

Vorteilhafterweise weist die Wärmerückgewinnungseinrichtung einen Wärmetauscher zum Betreiben des Brüdenverdichters auf, der zur Nutzung von Wärmeenergie von Abgas von Blockheizkraftwerken mit einer Abgasleitung eines Blockheizkraftwerkes verbunden bzw. verbindbar ist.

Gemäß einer besonderen Ausführungsform steht ein Ausgang des Brüdenverdichters mit einem radial äußeren unteren Bereich im Inneren der radial äußeren oder radial äußersten Überlaufwand zum Einleiten der verdichteten Gasphase in Fluidverbindung.

Insbesondere kann dabei vorgesehen sein, dass zwischen dem Ausgang des Brüdenverdichters und der radial äußeren oder radial äußersten Überlaufwand eine Mischdüse, insbesondere eine Ringmischdüse, geschaltet ist.

Schließlich kann vorgesehen sein, dass die Mischdüse einen zweiten Eingang aufweist, der mit dem unteren Bereich im Inneren der radial äußeren oder radial äußersten Überaufwand in Fluidverbindung steht oder bringbar ist.

Der vorliegenden Erfindung liegt die überraschende Erkenntnis zugrunde, dass durch die spezielle Verfahrensführung und/oder die spezielle Vorrichtung in einem integrierten System Gärreste etc. aufkonzentriert und das Abwasser direkt abgegeben werden kann.

Weitere Merkmale und Vorteile der Erfindung ergeben sich aus den beigefügten Ansprüchen und der nachfolgenden Beschreibung, in der besondere Ausführungsformen anhand der schematischen Zeichnungen im Einzelnen erläutert werden. Dabei zeigt:
- Figur 1: eine Vorrichtung zur Aufbereitung von biologischen Stoffen gemäß einer besonderen Ausführungsform der vorliegenden Erfindung teilweise in Vertikalschnittansicht;
- Figur 2: eine Schnittansicht entlang der Linie A-A' in Figur 1;
- Figur 3: Details einer in der Figur 1 schematisch gezeigten Ringmischdüse in Schnittansicht und in Seitenansicht ;
- Figur 4: eine Vorrichtung zur Aufbereitung von biologischen Stoffen gemäß einer weiteren besonderen Ausführungsform der vorliegenden Erfindung teilweise in Vertikalschnittansicht;
- Figur 5: Einzelheiten einer Vorrichtung zur Aufbereitung von biologischen Stoffen gemäß einer weiteren besonderen Ausführungsform der vorliegenden Erfindung; und
- Figur 6: ein Druck-Temperatur-Diagramm für das Ammoniak-Gleichgewicht.

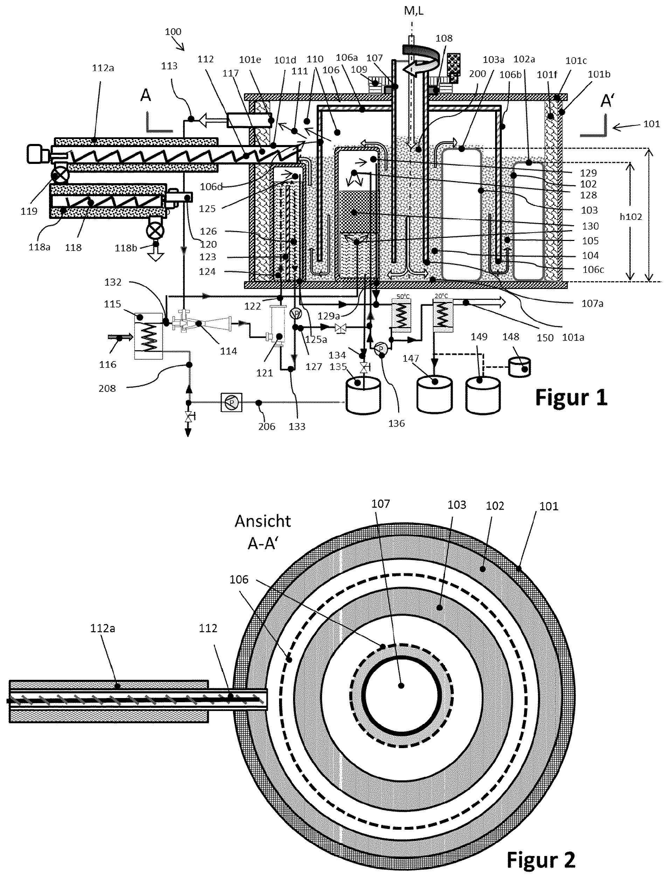

Die in den Figuren 1 bis 3 gezeigte Vorrichtung 100 umfasst eine Abtrenneinrichtung zur Abtrennung von Wasser und Stickstoffverbindungen, in diesem Beispiel Ammoniak, aus einem biologischen Stoff, in diesem Beispiel Gärreste 200. Die Abtrenneinrichtung weist einen im Grundriss runden Behälter 101 mit einem Behälterboden 101a, einer Behälterwand 101b und einen Behälterdeckel 101c, wobei im Behälter 101 zwei hohle zylindrische Überlaufwände 102 und 103 konzentrisch zur Mittelachse M des Behälters 101 unter Bildung einer im Grundriss runden Zuführkammer 104 und einer äußeren Ringkammer 105 angeordnet sind, wobei die Höhe der Überlaufkanten 102a bzw. 103a von der radial inneren Überlaufwand 103 zur radial äußeren Überlaufwand 102 abnimmt (Höhe h102 der Überlaufkante 102a < Höhe h103 der Überlaufkante 103a). Somit kann abhängig von der Zufördermenge eine Befüllung der Zuführkammer 104 und der Ringkammer 105 zwischen den Überlaufwänden 103 und 102 von innen nach außen erfolgen.

Die Überlaufwände 102 und 103 sind gegen den Behälterboden 101a abgedichtet.

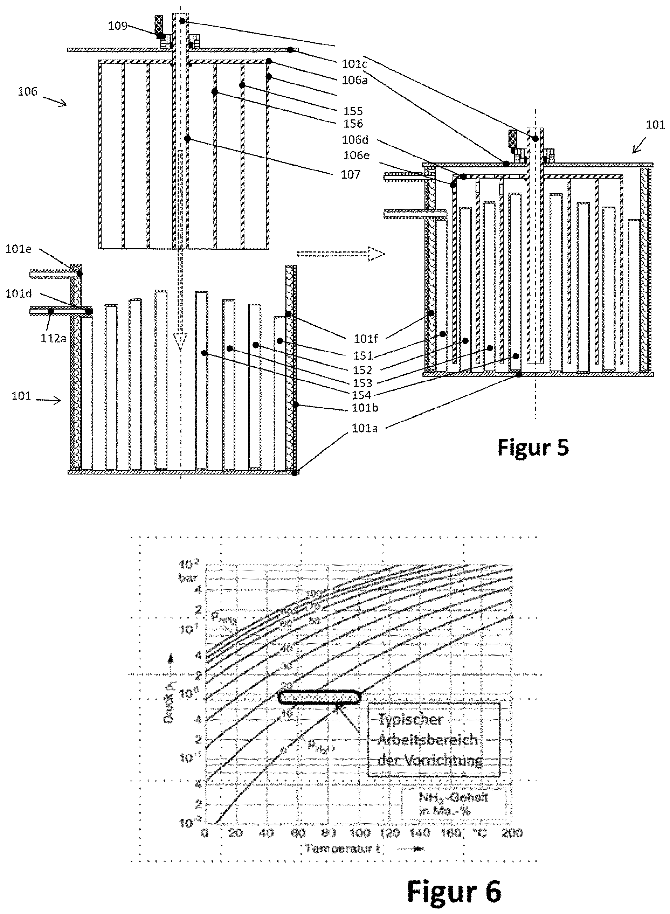

Wie in der Figur 5 gezeigt ist und später beschrieben wird, kann zur Leistungs-/Effizienzsteigerung die Anzahl der Überlaufwände beliebig erweitert bzw. vergrößert werden.

Des Weiteren umfasst die Abtrenneinrichtung eine im Behälter 101 konzentrisch angeordnete, drehbare Glocke 106 mit einer Glockenoberseite 106a, wobei die Glocke über die Überlaufwände 102 und 103 gestülpt ist und eine zylindrische Außenwand 106b aufweist, wobei die Unterseite 106c der Außenwand 106b der Glocke 106 oberhalb des Behälterbodens 101a endet und die Außenwand 106b der Glocke eine obere Auslassöffnung 106d aufweist, wobei der Behälter 101 in seiner Behälterwand 101b eine erste Behälterauslassöffnung 101d oberhalb der Überlaufkante 102a der radial äußeren Überlaufwand 102 sowie unterhalb der oberen Auslassöffnung 106d in der Außenwand 106b der Glocke 106 und eine zweite Behälterauslassöffnung 101e oberhalb der ersten Behälterauslassöffnung 101d aufweist, wobei die zweite Behälterauslassöffnung 101e mit der oberen Auslassöffnung 106d in der Außenwand 106b der Glocke 106 in Fluidverbindung steht. Die Auslassöffnung 106d kann auch weiter oberhalb bzw. im Glockendeckel 106a positioniert sein bzw. aus mehreren Öffnungen bestehen.

Ferner umfasst die Abtrenneinrichtung ein Befüllrohr 107, das sich von oben konzentrisch zur Mittelachse M des Behälters 101 durch den Behälterdeckel 101c und die Glockenoberseite 106a in den Behälter 101 hineinerstreckt, wobei die Unterseite 107a des Befüllrohres 107 oberhalb des Behälterbodens 101a endet und das Befüllrohr mit der Glocke 106, in diesem Beispiel fest, verbunden ist sowie im Behälterdeckel 101c um seine Längsachse L drehbar gelagert ist. Zudem ist das Befüllrohr 107 über eine Abdichtung 108, bspw. eine ringförmige Abdichtung, zum Behälterdeckel 101c abgedichtet. Damit kann das Innere des Behälters 101 nur über das Befüllrohr 107 mit der Atmosphäre in (Fluid)Verbindung gebracht werden.

Über einen Drehantrieb, zu dem in diesem Beispiel ein Drehkranz 109 gehört, der das Befüllrohr 107 oberhalb des Behälterdeckels 101c umgibt, kann das Befüllrohr 107 und damit die damit verbundene Glocke 106 um seine bzw. ihre Mittelachse gedreht werden. Über das Befüllrohr 107 kann die mittige Zuführkammer 104 mit bspw. den Gärresten befüllt werden. Das Befüllrohr 107 ragt so weit in die Zuführkammer 104 hinein, dass es über bspw. die Gärrestsäule eine Abdichtung zwischen dem oberen Bereich (Verdampfungsraum 110) und der Atmosphäre bildet. Über die Syphonwirkung des gefüllten Befüllrohres 107 wird der Behälter 101 gasdicht verschlossen.

Wenn die Glocke 106 über den Drehkranz 109 angetrieben wird, dann wirkt sie als Rührwerk für die Zwischenräume zwischen den Überlaufwänden 102 und 103 und dem Befüllrohr 107. Zudem gelangen durch die Drehung und die damit verbundenen Zentrifugalkräfte die Gärreste vom Befüllrohr 107 im Behälter 101 radial nach außen.

Des Weiteren weist die Abtrenneinrichtung eine Heizeinrichtung zum Erwärmen der Gärreste im Inneren des Behälters 101 vom Inneren der hohlen Überlaufwände 102 und 103 des Behälters 101 auf. Dazu werden die hohlen Überlaufwände 102 und 103 innen mit heißem Wasser und Dampf durchströmt. Die sich drehende Glocke 106 hält die Gärreste im Behälter 101 in Bewegung und verbessert damit den Wärmeübergang zwischen den Überlaufwänden 102 und 103 und den Gärresten. Genauer gesagt erfolgt ein Wärmeübergang von den Überlaufwänden 102 und 103 auf die Gärreste. Es erfolgt ein Verdampfen in der Form, dass die hohlen Überlaufwände 102 und 103 mit Wasser und Dampf befüllt sind und der Dampf bei höherem Dampfdruck (vorzugsweise 1300 mbar entspricht 107°C) und damit höheren Temperaturen kondensiert und die Kondensationsenergie über die Wandungen zum Gärrest gelangen kann und diesen bei niedrigem Druck (vorzugsweise atmosphärischer Druck von 1000 mbar entspricht 100°C) zum Verdampfen bringt. Durch die Kondensation stellt sich eine Wassertemperatur entsprechend dem Dampfdruck ein, der in dem Anwendungsfall bei vorzugsweise 107 °C oder auch 1300 mbar liegt. Es kondensiert in den Überlaufwänden immer nur die Menge an Dampf die zur Aufwärmung oder Verdampfung der Gärreste benötigt wird und die Temperatur des Heizwassers bleibt so lange bei 107°C, wie sich noch Dampf in den Überlaufwänden befindet. In der Zuführkammer 104 und in der Ringkammer 105 werden aufgrund der "beheizten" Überlaufwände 102 und 103 aus den Gärresten im wesentlichen Wasser und andere in den Gärresten gelöste Bestandteile, wie z.B. Ammoniak, verdampft. Der Dampf wird auch als Brüden 111 bezeichnet. Er sammelt sich in dem Verdampfungsraum 110 und wird über den Auslass 101e aus dem Behälter geführt. Die Gärreste konzentrieren sich von dem Bereich zwischen dem Befüllrohr 107 und der Überlaufwand 103 zur Ringkammer 105 auf.

Das Ganze wird über eine Steuereinrichtung (nicht gezeigt) zur Steuerung des Drehantriebs, der Heizeinrichtung und der Befüllung des Behälters gesteuert. Die Steuereinrichtung kann auch noch die Steuerung der übrigen Funktionen der Vorrichtung bzw. Komponente oder eines Teils davon übernehmen.

In diesem Beispiel ragt eine erste Fördereinrichtung in Form einer Förderspirale 112 von außen durch die erste Behälterauslassöffnung 101d in der Behälterwand 101b soweit hinein, dass über die erste Förderspirale die nunmehr konzentrierten überwiegend festen Gärreste abgefördert werden können. Die erste Förderspirale 112 ist darüber hinaus so ausgebildet bzw. mit entsprechenden Komponenten versehen, dass die konzentrierten Gärreste dem Behälter 101 entzogen werden und beispielsweise einer thermischen Nachtrocknung oder auch einer sonstigen Verwendung (direkte Nutzung als Dünger) zugeführt werden.

Der Verdampfungsraum 110 ermöglicht über die Auslassöffnung 106d in der Glocke 106 und die zweite Behälterauslassöffnung 101e in der Behälterwand 101b, dass mit der Verdampfung der Gärreste Luft (Sauerstoff und Stickstoff) den Verdampfungsraum 110 nach außen verlässt und kein neuer Sauerstoff über das als Syphon wirkende Befüllrohr 107 in den Behälter 101 gelangen kann. Ferner steht die zweite Behälterauslassöffnung 101e über eine Leitung 113, bspw. eine Rohrleitung, mit einem Dampfstrahlverdichter, wie z.B. einem Brüdenverdichter 114, in Fluidverbindung. Genauer gesagt wird der Dampf (Wasserdampf) bzw. Brüden 111 als Saugmedium dem Brüdenverdichter 114 zugeführt. Als Treibmedium für den Brüdenverdichter 114 wird vorzugsweise Wasserdampf verwendet, der bspw. über einen Wärmetauscher 115 erzeugt wird, wobei zur Erzeugung des Wasserdampfes in dem Wärmetauscher 115 vorzugsweise Abgas 116 bspw. von einem Blockheizkraftwerk (nicht gezeigt) verwendet wird.

Der Brüdenverdichter 114 ist Teil einer Wärmerückgewinnungseinrichtung zur Rückgewinnung von Wärmeenergie aus dem Brüden 111. Über den Brüdenverdichter 114 kann der Brüden 111 im Dampfdruck und damit der Kondensationstemperatur vergleichbar dem Wärmepumpeneffekt angehoben werden. Als Brüdenverdichter 114 kann auch ein mechanischer Verdichter (z.B. Radialgebläse bzw. Turbine) genutzt werden.

Das Abgas 117 der thermischen Nachtrocknung in der ersten Förderschnecke 112 kann ebenfalls dem Brüden 111 zugeführt werden, da in diesem Beispiel eine direkte Verbindung zum Verdampfungsraum 110 verbleibt.

Ferner weist die Vorrichtung 100 eine der ersten Förderspirale 112 nachgeschaltete Torrefizierungseinrichtung zur Torrefizierung des überwiegend festen Produkts der Nachtrocknung auf. Die Torrefizierungseinrichtung umfasst eine zweite Förderspirale 118, und das über den Trennprozess im Behälter 101 von Wasser und Sauerstoff überwiegend befreite Produkt (Feststoff) kann durch Einsatz höherer Temperaturen von bspw. ca. 300 °C in der zweiten Förderspirale 118 mit wenig Aufwand torrefiziert werden, da der Sauerstoff bereits im vorgeschalteten Trennprozess entzogen wurde.

Beim Torrefizieren wird Biomasse unter Sauerstoffabschluss bei ca. 250 °C bis ca. 400 °C aufgeheizt und es erfolgt eine teilweise pyrolytische Zersetzung unter Freisetzung von Wasser, Kohlenstoffdioxid, Kohlenstoffmonoxid und organischen Säuren. Durch Torrefizierung wird ein stabiler Wassergehalt von nur ca. 3 % erreicht. Die Masse wird um rund 30 % verringert, während der Energiegehalt nur um etwa 10 % abnimmt und raucherzeugende Stoffe (also Stoffe, die eher unvollständig unter anderem zu Ruß verbrennen würden) entfernt werden. Der Heizwert des schwarzen, krümeligen Produkts (auch Biokohle genannt) liegt bei ca. 19,9 bis ca. 22,7 MJ/kg (grünes, wasserhaltiges Holz hat ca. 10,5 - ca. 17,7 MJ/kg). Die Energie, die durch Erhöhung des Heizwerts "gewonnen" wird, wird aber zum Verdampfen des enthaltenen Wassers im Prozess benötigt. Das erhaltene Produkt kann leicht zu Pellets gepresst oder weiter zerkleinert werden.

Durch Druckstaffelung oder beispielsweise eine Schleuse 119 kann das belastete Abgas der Torrefizierung separiert werden und beispielsweise kann das Abgas über einen separierten Ausgang 120 einer anderen Verarbeitung (zum Beispiel Verbrennung) zugeführt werden.

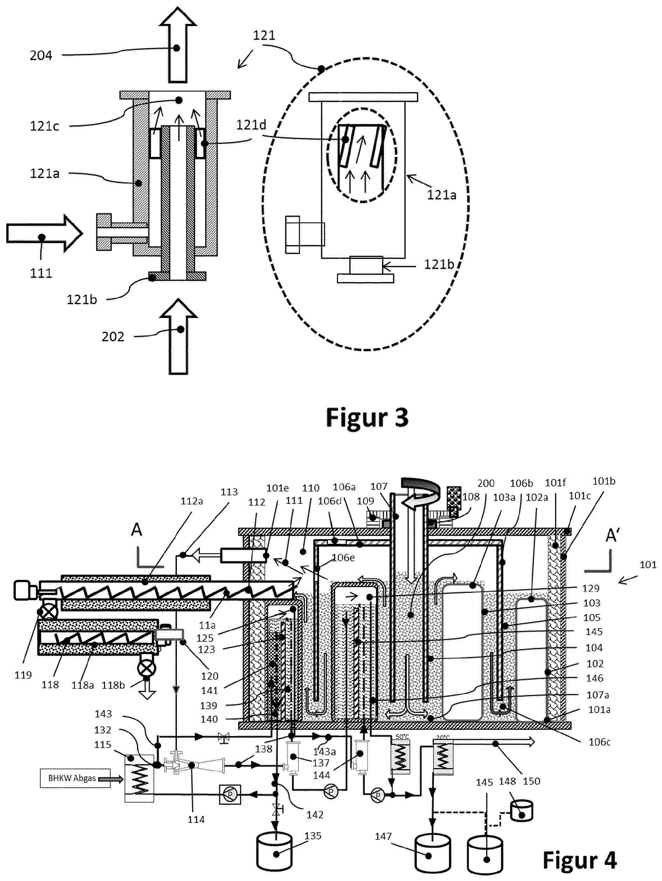

Durch eine unterschiedliche Beschaltung (siehe die in den Figuren 1 und 4 dargestellten Ausführungsbeispiele) der hohlen Überlaufwände 102 und 103 kann die Vorrichtung unterschiedliche Zusatzfunktionen erfüllen.

Der in dem Brüdenverdichter 114 verdichtete und in diesem Beispiel Ammoniak-haltige Brüden 111 wird über eine Ringmischdüse 121 und eine Leitung 122 in den äußeren und mit einem sich in diesem Beispiel in Schnittansicht vertikal erstreckendem Trennblech 123 im unteren Bereich abgetrennten Bereich 124 der hohlen Überlaufwand 102 eingespeist.

Wie sich aus der Figur 3 ergibt, besteht in diesem Beispiel die Ringmischdüse 121 im Wesentlichen aus zwei Hülsen 121a, 121b, die ineinander gesteckt sind. Der verdichtete Brüden 111 wird in die äußere Hülse 121a geleitet und ein zweites Medium 202, zum Beispiel Wasser, in die innere Hülse 121b. Am vorderen Ende der inneren Hülse 121b befindet sich in der äußeren Hülse 121a eine Mischkammer 121c. Beispielsweise durch am vorderen Ende der inneren Hülse 121b außen aufgesetzte Verwirbelungsbleche 121d wird der verdichtete Brüden 111 in Drehung versetzt und erhöht dadurch den Mischeffekt in der Mischkammer 121c. Das die Mischkammer verlassende "Mischmedium" 204 kann danach auch in den Transportleitungen weiter reagieren, bevor es in den nächsten Verarbeitungsschritt gelangt. Haben der Brüden 111 und das zweite Medium 202 unterschiedliche Geschwindigkeiten, dann wirkt das schnellere Medium für das langsamere Medium als Pumpe (Ejektor-Effekt).

Die Ringmischdüse 121 verbessert im Allgemeinen den Oberflächenkontakt zwischen den beiden Medien (Dampf (Brüden) und z.B. Wasser) und erleichtert die Abtrennung von beispielsweise Ammoniak (NH₃).

Im oberen Bereich 125 in der Überlaufwand 102 (s. Fig. 1) sammelt sich der nicht kondensierte und mit Ammoniak NH3 angereicherte Brüden und wird über die Leitung 125a abgeleitet. Der Grund für die teilweise Nichtkondensation liegt in dem Energieüberangebot bei dem eingestellten Taupunkt. Ein Teil des Kondensats 126 aus der Überlaufwand 102 wird über eine Leitung 127 und einen Sprühkopf 128 im oberen Bereich 129 oberhalb eines Stripping-Bereiches 130 in die Überlaufwand 103 eingesprüht. Gleichzeitig wird im Bereich 131 unterhalb des Stripping-Bereiches 130 in der Überlaufwand 103 Frischdampf 132 vom Wärmetauscher 115 eingelassen. Der übrige Teil des Kondensats 126 wird über die Leitung 133 als zweites Medium der Ringmischdüse 121 zugeführt und mit dem verdichteten Brüden 111 vermischt und der Überlaufwand 102 erneut zugeführt.

Das in der Überlaufwand 103 eingesprühte Wasser 128 wird über den Stripping-Bereich 130 und den Frischdampf 132 von Ammoniak befreit. Im Strippingbereich ist Oberflächen vergrößerndes Material (z.B. Eisenwolle) untergebracht und das Ammoniak wird über den Frischdampf dem Wasser entzogen. Das im Dampf konzentrierte Ammoniak sammelt sich im oberen Bereich 129 und wird von dort über die Leitung 129a abgeführt. Das gesäuberte Wasser wird über eine Leitung 134 einer Zwischenlagerung in Form eines Wassersammlers 135 und ggf. späteren Nutzung zugeführt. Dadurch dass ein Teil des Kondensats 126 aus der Überlaufwand 102 über die Ringmischdüse 121 noch einmal in der Überlaufwand 102 geführt wird, erfolgt in der Überlaufwand 102 eine mindestens 2-stufige Reaktion des Ammoniakhaltigen Dampfes und damit eine weitere Abtrennung von schwach Ammoniak-haltigem Wasser, das dann über eine Pumpe 136 dem Sprühkopf 128 zur endgültigen Ammoniak-Abtrennung zugeführt wird.

Das die innere Überlaufwand 103 über die Leitung 134 verlassende Wasser ist durch den vorgeschalteten Stripping-Prozess in dem Stripping-Bereich 130 soweit gereinigt, dass es in dem Wassersammler 135 gesammelt wird und für eine wirtschaftliche Weiternutzung (zum Beispiel Dampferzeugung (siehe die Leitungen 206 und 208)) zur Verfügung steht.

Hinsichtlich der Prozesse in den Überlaufwänden 102 und 103 ist noch zu ergänzen, dass sich das Ammoniak im Gleichgewicht befindet. Es ist teilweise in Wasser gelöst und teilweise gasförmig (zusammen mit Wasserdampf und ggf. anderen Gasen) oberhalb der Flüssigphase und wenn die Flüssigphase mit gelöstem Ammoniak abgetrennt wird (siehe auch Figur 6, in der der typische Arbeitsbereich der Vorrichtung gekennzeichnet ist) wird in der jeweiligen Überlaufwand wieder Ammoniak aus der Lösung in die Gasphase oberhalb der Flüssigkeit übergehen (je nach Temperatur, Druck und pH-Wert). Damit Ammoniak stabil in Lösungen verbleibt, nutzt man das Gleichgewicht:

NH₃ + H₃O⁺ **→** NH₄**⁺** + H₂O.

Die in der Figur 4 gezeigte Ausführungsform (wobei die Figur 2 ebenfalls die Schnittansicht entlang der Linie A-A' zeigt) einer Vorrichtung 100 unterscheidet sich von der in der Figur 1 gezeigten Vorrichtung 100 unter anderem darin, dass sich im Inneren der radial inneren Überlaufwand 103 kein Sprühkopf befindet. Zudem wird der mittels des Brüdenverdichters 114 verdichtete Brüden 111 über eine Ringmischdüse 137 mit einem weiter unten erläuterten Medium vermischt und über eine Leitung 138 in einen durch ein in diesem Beispiel sich in Schnittansicht vertikal ersteckendes Trennblech 123 getrennten unteren inneren Bereich 139 der radial äußeren Überlaufwand 102 geführt und wird in dem radial äußeren Bereich 140 der radial äußeren Überlaufwand 102 über einen Stripping-Bereich 141 über eine Leitung 142 zurückgeführt bzw. hinausgeführt. Frischdampf 132 wird dabei in Gegenströmung über eine Leitung 143 in den äußeren Bereich 140 der radial äußeren Überlaufwand 102 eingespeist. Der Frischdampf kann beispielsweise wie bei der Ausführungsform in den Figuren 1 bis 3 in dem Wärmetauscher 115 erzeugt werden. Das den Bereich 140 verlassende saubere Wasser wird über die Leitung 142 in den Sammler 135 gefördert oder auch einer anderen Nutzung wie z.B. Dampferzeugung zugeführt.

Der überschüssige Dampf im oberen Bereich 125 der radial äußeren Überlaufwand 102 wird über eine Leitung 143a und eine weitere Mischringdüse 144 mit zurückgeführtem Wasser vermischt und in einen durch ein sich in Schnittansicht in diesem Beispiel vertikal ersteckendes Trennblech 145 getrennten unteren radial inneren Bereich 146 der radial inneren Überlaufwand 103 zugeführt.

Bei der in der Figur 4 gezeigten Ausführungsform erfolgt die Kondensation des ammoniakhaltigen Dampfes von der radial inneren Überlaufwand 103 über zwei Kondensationsstufen. In der ersten Stufe mit höherer Temperatur (z.B. 50°C) wird schwach ammoniak-haltiges Wasser abgeschieden, welches dann über die Mischringdüse 144 erneut in die radial innere Überlaufwand 103 zur erneuten NH₃-Abreicherung gefördert wird. In der zweiten Kondensationsstufe (ca. 20°C) erfolgt die Restabscheidung mit hoher Ammoniak-Konzentration bis zu 20%.

Zudem wird bei der in der Figur 4 gezeigten Ausführungsform überschüssiges Wasser aus der radial inneren Überlaufwand 103 über die Mischringdüse 137 wieder der radial äußeren Überlaufwand 102 zur Ammoniak-Abtrennung zugeführt.

Bei den in den Figuren 1 bis 3 gezeigten Ausführungsformen der Vorrichtung wird gasförmiges Ammoniak nach der ersten Kondensationsstufe entweder über die zweite Kondensationsstufe auskondensiert und in einem Ammoniaksammler 147 gesammelt oder beispielsweise mit einer Säure, wie z.B. Schwefelsäure, in einem Behälter 148 zu Flüssigdünger in einem Behälter 149 vermischt und zur späteren Nutzung gelagert.

Alternativ kann das gasförmige Ammoniak direkt einer weiteren Nutzung, wie z.B. Verbrennung, zugeführt werden (siehe in Pfeil 150).

Bei beiden Ausführungsformen der Vorrichtung wird durch die Integration von Mischringdüsen grundsätzlich die Effizienz der Ammoniak-Abtrennung verbessert.

Durch eine Erhöhung der Anzahl der Überlaufwände (siehe die Überlaufwände 151 bis 154 in der Figur 5) und eine entsprechende Erhöhung der Anzahl der Ringwände (siehe die Ringwände 155 und 156 in der Figur 5) der Glocke 106 können weitere und ggf. effizientere Stripping-Optionen bspw. als Kombination aus Sprühen, Mischen und Druck- bzw. Temperaturveränderung zum Einsatz kommen.

Durch eine Erhöhung der Anzahl der Überlaufwände (siehe Figur 5) kann z.B. die innerste Überlaufwand zur Kühlung des gereinigten heißen Abwassers genutzt werden und damit die Energie zum Vorheizen der Gärreste eingesetzt werden.

Durch die Brüdenverdichtung hat der komplette Prozess nur einen sehr geringen Energiebedarf.

Weiterhin ist zu erwähnen, dass alle Prozesse (Abtrennung über Verdampfung, Wärmerückgewinnung über Brüdenverdichtung, Ammoniak-Abtrennung bspw. über Stripping, Ammoniak-Bindung bspw. zu Dünger) in einer Vorrichtung sehr kompakt integriert sind.

Als oberflächenvergrößerndes Stripping-Material können unterschiedlichste Materialien, wie z.B. Eisenwolle, Kies, Kugeln, Lochbleche, ... zum Einsatz kommen.

Der Prozess der Abtrennung kann auch für andere Abwässer (Industrieabwässer, Kläranlagen o.ä.) eingesetzt werden.

Zumindest in einer besonderen Ausführungsform kann mit den Vorrichtungen bspw. Ammoniak konzentriert und in einen nutzbringenden Wertstoff umgewandelt werden, der in der Industrie weiterverarbeitet werden kann zu bspw. Düngemittel, Reinigungsmittel, Brennstoff etc. Die Ammoniak-Abtrennung lässt sich mit dem Verfahren wirtschaftlich darstellen.

Der Energieverbrauch der Vorrichtung beläuft sich auf Bruchteile des Energieverbrauchs von normalen Trocknern, da der Wärmepumpeneffekt direkt über die Brüdenverdichtung realisiert wird und nur ein Druckanstieg von ca. 0,3 Bar (Taupunktanhebung von ca. 7 °C) erforderlich ist. Dies entspricht einer reinen Verdichterleistung von ca. 13 kW, um 1 m³ Wasser (Gärrest ist zu ca. 93% Wasser) pro h zu verdampfen. Setzt man einen mechanischen Verdichter ein, wird unter Berücksichtigung der Verlustleistung ca. 25 kW elektrische Leistung benötigt. Ist ein Blockheizkraftwerk vorhanden, kann das Abgas (ca. 250 kW bei einer 500 kWₑₗₑₖₜᵣ Block als Kraftwerk-Anlage) genutzt werden und könnte die Vorrichtung noch ca. 200 kW als Restwärme für andere Prozesse zur Verfügung stellen.

Zudem weist das abgetrennte Wasser einen geringen Nitrit- und Nitratgehalt auf und liegt es auch ansonsten im Wesentlichen innerhalb der Grenzwerte für Trinkwasser.

Der Ammoniakgehalt ist deutlich reduziert und kann durch die Verfahrensweise so beeinflusst werden, dass er innerhalb der Abwasser-Richtlinie liegt.

Torrefizierte Feststoffe sind durch die höhere Dichte gut zu lagern und zu transportieren und bilden als Biokohle einen guten CO₂-neutralen wertvollen Brennstoff mit relativ hohem Heizwert. Als Dünger oder Bodenverbesserer findet er Einsatz in Gärtnereien oder auch der normalen Landwirtschaft.

### Bezugszeichenliste

- 100: Vorrichtung
- 101: Behälter
- 101a: Behälterboden
- 101b: Behälterwand
- 101c: Behälterdeckel
- 101d: erste Behälterauslassöffnung
- 101e: zweite Behälterauslassöffnung
- 101f: Isolierung
- 102: Überlaufwand
- 102a: Überlaufkante
- 103: Überlaufwand
- 103a: Überlaufkante
- 104: Zuführkammer
- 105: Ringkammer
- 106: Glocke
- 106a: Glockenoberseite
- 106b: Außenwand
- 106c: Unterseite
- 106d: Auslassöffnung
- 106e: Durchlassöffnung
- 107: Befüllrohr
- 107a: Unterseite
- 108: Abdichtung
- 109: Drehkranz
- 110: Verdampfungsraum
- 111: Brüden
- 112: erste Förderspirale
- 112a: Förderrohr
- 113: Leitung
- 114: Brüdenverdichter
- 115: Wärmetauscher
- 116: Abgas
- 117: Abgas
- 118: zweite Förderspirale
- 118a: Förderrohr
- 118b: Feststoffausgang
- 119: Schleuse
- 120: Ausgang
- 121: Ringmischdüse
- 121a: Hülse
- 121b: Hülse
- 121c: Mischkammer
- 121d: Verwirbelungsbleche
- 122: Leitung
- 123: Trennblech
- 124: Bereich
- 125: oberer Bereich
- 125a: Leitung
- 126: Kondensat
- 127: Leitung
- 128: Sprühkopf
- 129: oberer Bereich
- 129a: Leitung
- 130: Stripping-Bereich
- 131: Bereich
- 132: Frischdampf
- 133: Leitung
- 134: Leitung
- 135: Wassersammler
- 136: Pumpe
- 137: Ringmischdüse
- 138: Leitung
- 139: innerer Bereich
- 140: äußerer Bereich
- 141: Stripping-Bereich
- 142: Leitung
- 143: Leitung
- 143a: Leitung
- 144: Mischringdüse
- 145: Trennblech
- 146: innerer Bereich
- 147: Ammoniak-Sammler
- 148: Behälter
- 149: Flüssigdünger
- 150: Pfeil
- 151-154: Überlaufwand
- 155-156: Ringwand
- 200: Gärreste
- 202: zweites Medium
- 204: Mischmedium
- 206: Leitung
- 208: Leitung
- h102: Höhe der Überlaufkante 102a
- h103: Höhe der Überlaufkante 103a
- L: Längsachse
- M: Mittelachse

## Patentansprüche

1. Vorrichtung (100) zur Aufbereitung von biologischen Stoffen, insbesondere von Gärresten (200) pflanzlicher und tierischer Herkunft, insbesondere aus Biogasanlagen, von Gülle oder von sonstigen organischen Abfallprodukten, enthaltend einen Feststoffanteil und einen flüssigen Anteil, wobei die Vorrichtung umfasst:
- eine Abtrenneinrichtung zur Abtrennung von Wasser und Stickstoffverbindungen, insbesondere Ammoniak, aus einem biologischen Stoff, wobei die Abtrenneinrichtung umfasst:
- einen im Grundriss runden Behälter (101; 148) mit einem Behälterboden (101a), einer Behälterwand (101b) und einem Behälterdeckel (101c), wobei im Behälter mindestens zwei hohle zylindrische Überlaufwände (102, 103; 151, 152, 153, 154) konzentrisch zur Mittelachse M des Behälters unter Bildung einer im Grundriss runden Zuführkammer (104) und mindestens einer äußeren Ringkammer (105) angeordnet sind, wobei die Höhe h 102, h 103 der Überlaufkanten (102a, 103a) der Überlaufwände (102, 103) von Überlaufkante zu Überlaufkante von innen nach außen abnimmt,
- eine im Behälter (101; 148) konzentrisch angeordnete, drehbare Glocke (106) mit einer Glockenoberseite (106a), wobei die Glocke über die Überlaufwände (102, 103; 151-154) gestülpt ist und eine zylindrische Außenwand (106b) und ggf. mindestens eine konzentrisch angeordnete radial innere zylindrische Ringwand (155, 156), die von oben in die mindestens eine Ringkammer (105) greift, aufweist, wobei die Unterseite(n) (106c) der Außenwand (106b) und der ggf. vorhandenen mindestens einen Ringwand (155, 156) der Glocke (106) oberhalb des Behälterbodens (101a) endet/enden und die Außenwand (106) und/oder die Glockenoberseite (106a) der Glocke eine obere Auslassöffnung (106d) und die ggf. vorhandene mindestens eine Ringwand der Glocke eine obere Durchlassöffnung (106e) aufweisen, wobei der Behälter in seiner Behälterwand eine erste Behälterauslassöffnung (101d) oberhalb der Überlaufkante (102a) der radial äußeren bzw. radial äußersten Überlaufwand (102) sowie unterhalb der mindestens einen oberen Auslassöffnung (106d) in der Außenwand (106b) der Glocke (106) und eine zweite Behälterauslassöffnung (101e) oberhalb der ersten Behälterauslassöffnung (101d) aufweist, wobei die zweite Behälterauslassöffnung (101e) mit der mindestens einen oberen Auslassöffnung (106d) in der Außenwand (106b) der Glocke (106) in Fluidverbindung steht,
- ein Befüllrohr (107), das sich von oben konzentrisch zur Mittelachse M des Behälters (101; 148) durch den Behälterdeckel (101c) und die Glockenoberseite (106a) in den Behälter (101; 148) hinein erstreckt, wobei die Unterseite (107a) des Befüllrohres (107) oberhalb des Behälterbodens (101a) endet und das Befüllrohr (107) mit der Glocke (106), insbesondere fest, verbunden ist sowie im Behälterdeckel (101c) um seine Längsachse L drehbar gelagert ist,
- eine Abdichtung (108) zum Abdichten des Befüllrohres (107) und des Behälterdeckels (101c),
- einen Drehantrieb zum Drehen des Befüllrohres (107) um seine Längsachse L, und
- eine Heizeinrichtung zum Erwärmen des biologischen Stoffes im Inneren des Behälters (101; 148) vom Inneren der hohlen Überlaufwände (102, 103; 151-154) des Behälters, (101) insbesondere auf eine Temperatur ca. von 100 °C, insbesondere bei einem atmosphärischen Druck von ca. 1 bar, zur Erzeugung einer hinsichtlich des Feststoffanteils aufkonzentrierten Flüssigphase und einer Wasser und Stickstoffverbindungen, insbesondere Ammoniak, enthaltenden Gasphase im Inneren des Behälters (101; 148).

2. Vorrichtung (100) nach Anspruch 1, wobei sie eine Steuereinrichtung zur Steuerung des Drehantriebs und/oder der Heizeinrichtung und/oder der Befüllung des Behälters (101; 148) und/oder der Abführung der aufkonzentrierten Flüssigphase und/oder der Gasphase aufweist.

3. Vorrichtung (100) nach Anspruch 1 oder 2, wobei zum Erwärmen des biologischen Stoffes im Inneren des Behälters (101; 148) die hohlen zylindrischen Überlaufwände (102, 103) mit Wasser und Dampf befüllt sind.

4. Vorrichtung (100) nach einem der Ansprüche 1 bis 3, wobei sie eine erste Fördereinrichtung zum Abfördern der aufkonzentrierten Flüssigphase aufweist, die mit der ersten Behälterauslassöffnung (101d) in Fluidverbindung steht.

5. Vorrichtung (100) nach Anspruch 4, wobei die erste Fördereinrichtung eine Förderspirale (112) umfasst.

6. Vorrichtung (100) nach Anspruch 4 oder 5, wobei die erste Fördereinrichtung zusätzlich zur thermischen Nachtrocknung der aufkonzentrierten Flüssigphase ausgebildet ist.

7. Vorrichtung (100) nach einem der Ansprüche 5 oder 6, wobei sie eine der ersten Fördereinrichtung nachgeschaltete Torrefizierungseinrichtung zur Torrefizierung des überwiegend festen Produkts der Nachtrocknung aufweist, insbesondere wobei die Torrefizierungseinrichtung eine der ersten Fördereinrichtung nachgeschaltete zweite Fördereinrichtung, insbesondere eine Förderspirale (118), aufweist.

8. Vorrichtung (100) nach einem der Ansprüche 1 bis 7, wobei sie eine Stickstoffverbindungen-Abtrenneinrichtung aufweist, die zur Abtrennung von Stickstoffverbindungen, wie z.B. Ammoniak oder Ammonium, aus der Gasphase mit der zweiten Behälterauslassöffnung (101e) direkt oder indirekt in Fluidverbindung steht.

9. Vorrichtung (100) nach Anspruch 8, wobei die Stickstoffverbindungen-Abtrenneinrichtung eine Separiereinrichtung zur Separierung eines Wasseranteils durch Kondensation von Wasser aus der Gasphase in der radial äußeren oder radial äußersten Überlaufwand (102) aufweist.

10. Vorrichtung (100) nach Anspruch 8 oder 9, wobei die Stickstoffverbindungen-Abtrenneinrichtung eine Stripping-Einrichtung zum Stickstoffverbindungen-Stripping in der radial inneren Überlaufwand (103) oder in mindestens einer der radial inneren Überlaufwände aufweist.

11. Vorrichtung (100) nach einem der vorangehenden Ansprüche, wobei sie eine Stickstoffverbindungen-Bindeeinrichtung zur Erzeugung von Flüssigdünger aus den abgetrennten Stickstoffverbindungen aufweist.

12. Vorrichtung (100) nach einem der Ansprüche 1 bis 11, wobei sie eine Ausleiteinrichtung zum Ausleiten von aufkonzentrierten Stickstoffverbindungen zur Weiterverarbeitung aufweist.

13. Vorrichtung (100) nach einem der Ansprüche 1 bis 12, wobei sie eine Wärmerückgewinnungseinrichtung zur Rückgewinnung von Wärmeenergie der Gasphase aufweist.

14. Vorrichtung (100) nach Anspruch 13, wobei die Wärmerückgewinnungseinrichtung einen Brüdenverdichter (114) aufweist, der mit der zweiten Behälterauslassöffnung (101e) zur Verdichtung der Gasphase in Fluidverbindung steht.

15. Vorrichtung (100) nach Anspruch 14, wobei die Wärmerückgewinnungseinrichtung einen Wärmetauscher (115) zum Betreiben des Brüdenverdichters (114) aufweist, der zur Nutzung von Wärmeenergie von Abgas von Blockheizkraftwerken mit einer Abgasleitung eines Blockheizkraftwerkes verbunden bzw. verbindbar ist.

16. Vorrichtung (100) nach Anspruch 14 oder 15, wobei ein Ausgang des Brüdenverdichters (114) mit einem radial äußeren unteren Bereich im Inneren der radial äußeren oder radial äußersten Überlaufwand (102) zum Einleiten der verdichteten Gasphase in Fluidverbindung steht.

17. Vorrichtung (100) nach Anspruch 16, wobei zwischen dem Ausgang des Brüdenverdichters (114) und der radial äußeren oder radial äußersten Überlaufwand (102) eine Mischdüse, insbesondere eine Ringmischdüse (137), geschaltet ist, insbesondere wobei die Mischdüse einen zweiten Eingang aufweist, der mit dem unteren Bereich im Inneren der radial äußeren oder radial äußersten Überlaufwand (102) in Fluidverbindung steht oder bringbar ist.

18. Verfahren zur Aufbereitung von biologischen Stoffen, insbesondere von Gärresten pflanzlicher und tierischer Herkunft, insbesondere aus Biogasanlagen, von Gülle oder von sonstigen organischen Abfallprodukten, enthaltend einen Feststoffanteil und einen flüssigen Anteil, unter Verwendung einer Vorrichtung nach einem der vorangehenden Ansprüche, wobei das Verfahren die folgenden Schritte umfasst:
- Befüllen des Behälters (101; 148) mittels des Befüllrohres (107) mit einem biologischen Stoff,
- vorzugsweise gleichzeitiges Drehen der Glocke (106),
- Abtrennen von Wasser und Stickstoffverbindungen, insbesondere Ammoniak, aus dem biologischen Stoff in Form von Brüden mittels Verdampfung,
- Rückgewinnen von Wärme aus dem Brüden mittels Brüdenverdichtung, und
- Abtrennen der Stickstoffverbindungen, insbesondere Ammoniak, aus dem verdichteten Brüden mittels Stripping und Separierung des Wasseranteils.

19. Verfahren nach Anspruch 18, ferner umfassend Auskondensation von gasförmigen Stickstoffverbindungen, insbesondere Ammoniak, nach einer ersten Kondensationsstufe zur Kondensation des Stickstoffverbindungen enthaltenden Brüden in einer zweiten Kondensationsstufe.

20. Verfahren nach Anspruch 19, ferner umfassend nachfolgendes Sammeln der auskondensierten Stickstoffverbindungen, insbesondere Ammoniak, in einer Sammeleinrichtung, insbesondere einem Ammoniaksammler.

21. Verfahren nach Anspruch 19, ferner umfassend Vermischen der auskondensierten Stickstoffverbindungen, insbesondere Ammoniak, mit einer Säure zu Flüssigdünger, insbesondere umfassend Lagern des Flüssigdüngers zur späteren Nutzung.

22. Verfahren nach Anspruch 18, ferner umfassend Kondensation des Stickstoffverbindungen enthaltenden Brüden in einer ersten Kondensationsstufe und Ausleiten von gasförmigen Stickstoffverbindungen, insbesondere Ammoniak, zur Weiterverarbeitung.

## Claims

1. Apparatus (100) for treatment of biological materials, especially of digestates (200) of vegetable and animal origin, especially from biogas plants, of liquid manure or of other organic waste products, containing a solids fraction and a liquid fraction, wherein the apparatus comprises:
- a separating means for separating water and nitrogen compounds, especially ammonia, from a biological material, wherein the separating means comprises:
- a container (101; 148) which is round in plan view having a container floor (101a), a container wall (101b) and a container lid (101c), wherein in the container at least two hollow cylindrical overflow walls (102, 103; 151, 152, 153, 154) are arranged concentrically with the central axis M of the container to form a feed chamber (104) which is round in plan view and at least one outer ring chamber (105), wherein the height h 102, h 103 of the overflow edges (102a, 103a) of the overflow walls (102, 103) decreases from inside to outside from overflow edge to overflow edge,
- a rotatable bell (106) arranged concentrically in the container (101; 148) having a bell top surface (106a), wherein the bell is placed over the overflow walls (102, 103; 151-154) and has a cylindrical outer wall (106b) and optionally at least one concentrically arranged radially inner cylindrical ring wall (155, 156) which reaches from above into the at least one ring chamber (105), wherein the bottom surface(s) (106c) of the outer wall (106b) and the optionally present at least one ring wall (155, 156) of the bell (106) terminate(s) above the container floor (101a) and the outer wall (106) and/or the bell top surface (106a) of the bell has/have an upper outlet opening (106d) and the optionally present at least one ring wall of the bell has an upper throughflow opening (106e), wherein the container in its container wall has a first container outlet opening (101d) above the overflow edge (102a) of the radially outer/radially outermost overflow wall (102) and below the at least one upper outlet opening (106d) in the outer wall (106b) of the bell (106) and a second container outlet opening (101e) above the first container outlet opening (101d), wherein the second container outlet opening (101e) is in fluidic communication with the at least one upper outlet opening (106d) in the outer wall (106b) of the bell (106),
- a fill pipe (107) which extends from above concentrically with the central axis M of the container (101; 148) through the container lid (101c) and the bell top surface (106a) into the container (101; 148), wherein the bottom surface (107a) of the fill pipe (107) terminates above the container floor (101a) and the fill pipe (107) is, especially fixedly, connected to the bell (106) and is mounted rotatably about its longitudinal axis L in the container lid (101c),
- a seal (108) for sealing the fill pipe (107) and the container lid (101c),
- a rotary drive for rotating the fill pipe (107) about its longitudinal axis L, and
- a heating means for heating the biological material inside the container (101; 148) from inside the hollow overflow walls (102, 103; 151-154) of the container (101), especially to a temperature of about 100°C, especially at an atmospheric pressure of about 1 bar, to produce a liquid phase concentrated with respect to the solids content and a gas phase containing water and nitrogen compounds, especially ammonia, inside the container (101; 148).

2. Apparatus (100) according to Claim 1, wherein said apparatus has a control means for controlling the rotary drive and/or the heating means and/or the filling of the container (101; 148) and/or the discharging of the concentrated liquid phase and/or the gas phase.

3. Apparatus (100) according to Claim 1 or 2, wherein the hollow cylindrical overflow walls (102, 103) are filled with water and steam for heating the biological material inside the container (101; 148).

4. Apparatus (100) according to any of Claims 1 to 3, wherein said apparatus comprises a first conveying means for discharging the concentrated liquid phase which is in fluidic communication with the first container outlet opening (101d).

5. Apparatus (100) according to Claim 4, wherein the first conveying means comprises a conveying screw (112).

6. Apparatus (100) according to Claim 4 or 5, wherein the first conveying means is implemented in addition to the thermal post-drying of the concentrated liquid phase.

7. Apparatus (100) according to either of Claims 5 or 6, wherein said apparatus comprises a torrefaction means arranged downstream of the first conveying means for torrefaction of the predominantly solid product of the post-drying, especially wherein the torrefaction means comprises a second conveying means, especially a conveying screw (118), arranged downstream of the first conveying means.

8. Apparatus (100) according to any of Claims 1 to 7, wherein said apparatus comprises a nitrogen compounds separating means which is in direct or indirect fluidic communication with the second container outlet opening (101e) for separation of nitrogen compounds, for example ammonia or ammonium, from the gas phase.

9. Apparatus (100) according to Claim 8, wherein the nitrogen compounds separating means has a separation means for separating a water fraction by condensation of water from the gas phase in the radially outer or radially outermost overflow wall (102).

10. Apparatus (100) according to Claim 8 or 9, wherein the nitrogen compounds separating means comprises a stripping means for nitrogen compounds stripping in the radially inner overflow wall (103) or in at least one of the radially inner overflow walls.

11. Apparatus (100) according to any of the preceding claims, wherein said apparatus comprises a nitrogen compounds fixing means for producing liquid fertilizer from the separated nitrogen compounds.

12. Apparatus (100) according to any of Claims 1 to 11, wherein said apparatus comprises a discharging means for discharging concentrated nitrogen compounds for further processing.

13. Apparatus (100) according to any of Claims 1 to 12, wherein said apparatus comprises a thermal recovery means for recovering thermal energy from the gas phase.

14. Apparatus (100) according to Claim 13, wherein the thermal recovery means comprises a vapour compressor (114) which is in fluidic communication with the second container outlet opening (101e) for compressing the gas phase.

15. Apparatus (100) according to Claim 14, wherein the thermal recovery means comprises a heat exchanger (115) for operating the vapour compressor (114) which is connected or connectable to an offgas conduit of a block heat and power plant to utilize thermal energy from offgas from block heat and power plants.

16. Apparatus (100) according to Claim 14 or 15, wherein an outflow of the vapour compressor (114) is in fluidic communication with a radially outer lower region inside the radially outer or radially outermost overflow wall (102) for introducing the compressed gas phase.

17. Apparatus (100) according to Claim 16, wherein a mixing nozzle, especially a ring mixing nozzle (137), is connected between the outflow of the vapour compressor (114) and the radially outer or radially outermost overflow wall (102), especially wherein the mixing nozzle has a second inflow which is in or can be brought into fluidic communication with the lower region inside the radially outer or radially outermost overflow wall (102).

18. Process for treatment of biological materials, especially of digestates of vegetable and animal origin, especially from biogas plants, of liquid manure or of other organic waste products, containing a solids fraction and a liquid fraction, using an apparatus according to any of the preceding claims, wherein the process comprises the steps of:
- filling the container (101; 148) with a biological material by means of the fill pipe (107),
- preferably simultaneously rotating the bell (106),
- separating water and nitrogen compounds, especially ammonia, from the biological material in the form of vapour by evaporation,
- recovering heat from the vapour by vapour compression, and
- separating the nitrogen compounds, especially ammonia, from the compressed vapour by stripping and separation of the water fraction.

19. Process according to Claim 18, further comprising condensing out gaseous nitrogen compounds, especially ammonia, after a first condensation stage for condensation of the vapour containing nitrogen compounds in a second condensation stage.

20. Process according to Claim 19, further comprising subsequently collecting the condensed-out nitrogen compounds, especially ammonia, in a collecting means, especially an ammonia collector.

21. Process according to Claim 19, further comprising mixing the condensed-out nitrogen compounds, especially ammonia, with an acid to afford liquid fertilizer, especially comprising storing the liquid fertilizer for later use.

22. Process according to Claim 18, further comprising condensing the vapour containing nitrogen compounds in a first condensation stage and discharging gaseous nitrogen compounds, especially ammonia, for further processing.

## Revendications

1. Dispositif (100) de traitement de substances biologiques, en particulier de résidus de fermentation (200) d'origine végétale et animale, en particulier provenant d'installations de biogaz, de lisier ou d'autres déchets organiques, contenant une fraction solide et une fraction liquide, le dispositif comprenant :
- un système de séparation destiné à séparer l'eau et les composés azotés, en particulier l'ammoniac, d'une substance biologique, le système de séparation comprenant :
- un contenant (101 ; 148) rond en plan avec un fond (101a) de contenant, une paroi (101b) de contenant et un couvercle (101c) de contenant, au moins deux parois de trop-plein cylindriques creuses (102, 103 ; 151, 152, 153, 154) étant disposées concentriquement à l'axe central M du contenant en formant une chambre d'alimentation (104) ronde en plan et au moins une chambre annulaire extérieure (105), la hauteur h 102, h 103 des bords de trop-plein (102a, 103a) des parois de trop-plein (102, 103) diminuant de l'intérieur vers l'extérieur d'un bord de trop-plein à l'autre,
- une cloche rotative (106) disposée concentriquement dans le contenant (101 ; 148) avec une face supérieure (106a) de cloche, la cloche étant retournée sur les parois de trop-plein (102, 103 ; 151-154) et comportant une paroi extérieure cylindrique (106b) et éventuellement au moins une paroi annulaire (155, 156) cylindrique radialement intérieure radiale disposée concentriquement, qui vient en prise depuis le haut avec l'au moins une chambre annulaire (105), la ou les faces inférieures (106c) de la paroi extérieure (106b) et de l'au moins une paroi annulaire (155, 156) éventuellement présente de la cloche (106) se terminant au-dessus du fond (101a) de contenant et la paroi extérieure (106) et/ou la face supérieure (106a) de cloche de la cloche comportant une ouverture de sortie supérieure (106d) et l'au moins une paroi annulaire éventuellement présente de la cloche comportant une ouverture de passage supérieure (106e), le contenant comportant dans sa paroi de contenant une première ouverture de sortie (101d) de contenant au-dessus du bord de trop-plein (102a) de la paroi de trop-plein radialement extérieure ou radialement la plus extérieure (102) et au-dessous de l'au moins une ouverture de sortie supérieure (106d) dans la paroi extérieure (106b) de la cloche (106) une deuxième ouverture de sortie (101e) de contenant au-dessus de la première ouverture de sortie (101d) de contenant, la deuxième ouverture de sortie (101e) de contenant étant en communication fluidique avec l'au moins une ouverture de sortie supérieure (106d) dans la paroi extérieure (106b) de la cloche (106),
- un tube de remplissage (107), qui s'étend depuis le haut concentriquement à l'axe central M du contenant (101 ; 148) à travers le couvercle (101c) de contenant et la face supérieure (106a) de cloche à l'intérieur du contenant (101 ; 148), la face inférieure (107a) du tube de remplissage (107) se terminant au-dessus du fond (101a) de contenant et le tube de remplissage (107) étant relié, en particulier fixement, à la cloche (106) et étant monté de manière à pouvoir tourner autour de son axe longitudinal L dans le couvercle (101c) de contenant,
- un joint (108) destiné à étanchéifier le tube de remplissage (107) et le couvercle (101c) de contenant,
- un entraînement rotatif destiné à faire tourner le tube de remplissage (107) autour de son axe longitudinal L, et
- un système de chauffage destiné à chauffer la substance biologique à l'intérieur du contenant (101 ; 148) depuis l'intérieur des parois de trop-plein creuses (102, 103 ; 151-154) du contenant (101), en particulier à une température d'environ 100 °C, en particulier à une pression atmosphérique d'environ 1 bar, pour générer une phase liquide concentrée en termes de fraction solide et une phase gazeuse contenant de l'eau et des composés azotés, en particulier de l'ammoniac, à l'intérieur du contenant (101 ; 148).

2. Dispositif (100) selon la revendication 1, comportant un système de commande destiné à commander l'entraînement rotatif et/ou le système de chauffage et/ou le remplissage du contenant (101 ; 148) et/ou l'évacuation de la phase liquide concentrée et/ou de la phase gazeuse.

3. Dispositif (100) selon la revendication 1 ou 2, les parois de trop-plein cylindriques creuses (102, 103) étant remplies d'eau et de vapeur pour chauffer la substance biologique à l'intérieur du contenant (101 ; 148).

4. Dispositif (100) selon l'une des revendications 1 à 3, comportant un premier système de transport destiné à évacuer la phase liquide concentrée, lequel est en communication fluidique avec la première ouverture de sortie (101d) de contenant.

5. Dispositif (100) selon la revendication 4, le premier système de transport comprenant une spirale de transport (112).

6. Dispositif (100) selon la revendication 4 ou 5, le premier système de transport étant formé en plus pour le séchage thermique ultérieur de la phase liquide concentrée.

7. Dispositif (100) selon l'une des revendications 5 ou 6, comportant un système de torréfaction placé en aval du premier système de transport, destiné à torréfier le produit majoritairement solide du séchage ultérieur, en particulier le système de torréfaction comportant un deuxième système de transport, en particulier une spirale de transport (118), placé en aval du premier système de transport.

8. Dispositif (100) selon l'une des revendications 1 à 7, comportant un système d'élimination de composés azotés qui, pour éliminer des composés azotés, par exemple de l'ammoniac ou de l'ammonium, de la phase gazeuse, est en communication fluidique directe ou indirecte avec la deuxième ouverture de sortie (101e) de contenant.

9. Dispositif (100) selon la revendication 8, le système de séparation de composés azotés comportant un système de séparation destiné à séparer une fraction d'eau par condensation d'eau de la phase gazeuse dans la paroi de trop-plein radialement extérieure ou radialement la plus extérieure (102).

10. Dispositif (100) selon la revendication 8 ou 9, le système de séparation de composés azotés comportant un système de stripping pour le stripping de composés azotés dans la paroi de trop-plein radialement intérieure (103) ou dans au moins une des parois de trop-plein radialement intérieures.

11. Dispositif (100) selon l'une des revendications précédentes, comportant un système de liaison de composés azotés pour générer un engrais liquide à partir des composés azotés séparés.

12. Dispositif (100) selon l'une des revendications 1 à 11, comportant un système de décharge pour décharger des composés azotés concentrés pour un traitement ultérieur.

13. Dispositif (100) selon l'une des revendications 1 à 12, comportant un système de récupération de chaleur destiné à récupérer de l'énergie thermique de la phase gazeuse.

14. Dispositif (100) selon la revendication 13, le système de récupération de chaleur comportant un compresseur de vapeur (114), qui est en communication fluidique avec la deuxième ouverture de sortie (101e) du contenant pour comprimer la phase gazeuse.

15. Dispositif (100) selon la revendication 14, le système de récupération de chaleur comportant un échangeur de chaleur (115) pour faire fonctionner le compresseur de vapeur (114), lequel est relié ou peut être relié à une ligne de gaz d'échappement d'une centrale de cogénération pour utiliser l'énergie thermique provenant de gaz d'échappement de centrales de cogénération.

16. Dispositif (100) selon la revendication 14 ou 15, une sortie du compresseur de vapeur (114) étant en communication fluidique avec une zone inférieure radialement extérieure à l'intérieur de la paroi de trop-plein radialement extérieure ou radialement la plus extérieure (102) pour l'introduction de la phase gazeuse comprimée.

17. Dispositif (100) selon la revendication 16, une buse de mélange, en particulier une buse de mélange annulaire (137), étant placée entre la sortie du compresseur de vapeur (114) et la paroi de trop-plein radialement extérieure ou radialement la plus extérieure (102), en particulier la buse de mélange comportant une deuxième entrée, qui est ou peut être amenée en communication fluidique avec la zone inférieure à l'intérieur de la paroi de trop-plein radialement extérieure ou radialement la plus extérieure (102).

18. Procédé de traitement de substances biologiques, en particulier de résidus de fermentation d'origine végétale et animale, en particulier d'installations de biogaz, de lisier ou d'autres déchets organiques, contenant une fraction solide et une fraction liquide, en utilisant un dispositif selon l'une des revendications précédentes, le procédé comprenant les étapes suivantes :
- remplissage du contenant (101 ; 148) avec une substance biologique au moyen du tube de remplissage (107),
- de préférence rotation simultanée de la cloche (106),
- séparation de l'eau et de composés azotés, en particulier de l'ammoniac, de la substance biologique sous forme de vapeurs par évaporation,
- récupération de la chaleur de la vapeur par compression de vapeur, et
- séparation des composés azotés, en particulier de l'ammoniac, de la vapeur comprimée par stripping et séparation de la fraction aqueuse.

19. Procédé selon la revendication 18, comprenant en outre une extraction par condensation de composés azotés gazeux, en particulier d'ammoniac, après une première étape de condensation pour condenser de la vapeur contenant des composés azotés dans une deuxième étape de condensation.

20. Procédé selon la revendication 19, comprenant en outre une collecte ultérieure des composés azotés extraits par condensation, en particulier de l'ammoniac, dans un système de collecte, en particulier un système de collecte d'ammoniac.

21. Procédé selon la revendication 19, comprenant en outre le mélange des composés azotés extraits par condensation, en particulier de l'ammoniac, avec un acide pour obtenir un engrais liquide, comprenant en particulier le stockage de l'engrais liquide pour une utilisation ultérieure.

22. Procédé selon la revendication 18, comprenant en outre la condensation de vapeur contenant des composés azotés dans une première étape de condensation et l'évacuation de composés azotés gazeux, en particulier de l'ammoniac, pour un traitement ultérieur.
